Europäisches Patentamt
European Patent Office
Office européen des brevets

(11) Veröffentlichungsnummer: **0 014 676**
**A2**

(12) **EUROPÄISCHE PATENTANMELDUNG**

(21) Anmeldenummer: 80810022.6

(22) Anmeldetag: 28.01.80

(51) Int. Cl.³: C 07 C 127/22
A 01 N 47/34

(30) Priorität: 01.02.79 CH 984/79
25.10.79 CH 9587/79

(43) Veröffentlichungstag der Anmeldung:
20.08.80 Patentblatt 80/17

(84) Benannte Vertragsstaaten:
BE CH DE FR GB IT NL

(71) Anmelder: CIBA-GEIGY AG
Patentabteilung Postfach
CH-4002 Basel(CH)

(72) Erfinder: Ehrenfreund, Josef, Dr.
Langenhagweg 11
CH-4123 Allschwil(CH)

(54) **Substituierte N-Phenyl-N'-benzoylharnstoffe, Verfahren zu ihrer Herstellung, diese Verbindungen enthaltende Mittel und ihre Verwendung zur Bekämpfung von Schädlingen.**

(57) Neue halogensubstituierte N-Phenyl-N'-bezoylharnstoffe der Formel

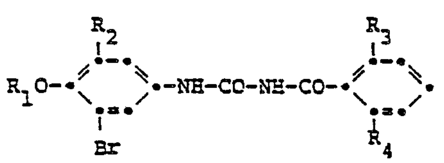

worin $R_1$ einen $C_2$-$C_3$-Alkenylrest oder einen einfach mit Chlor substituierten $C_2$-$C_3$-Alkenylrest; $R_2$ und $R_3$ Halogen; und $R_4$ Wasserstoff oder Halogen bedeuten, Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen besitzen ferner frasshemmende Wirkung gegenüber pflanzenschädigenden Insekten und sind teilweise ovizid und larvizid wirksam.

EP 0 014 676 A2

Croydon Printing Company Ltd.

0014676



BEZEICHNUNG GEÄNDERT
siehe Titelseite

CIBA-GEIGY AG
Basel (Schweiz

5-12211/1+2/+

Phenylharnstoffe

Die vorliegende Erfindung betrifft neue halogensubstituierte N-Phenyl-N'-benzoylharnstoffe, Verfahren zu ihrer Herstellung und Ihre Verwendung in der Schädlingsbekämpfung.

Die erfindungsgemässen halogensubstituierten N-Phenyl-N'-benzoylharnstoffe haben die Formel I

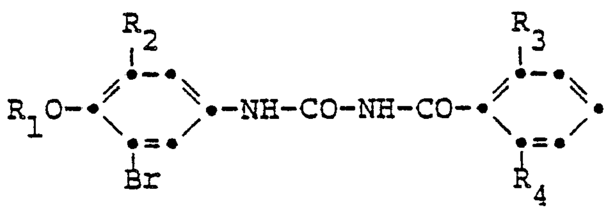

(I),

worin $R_1$ einen $C_2$-$C_3$-Alkenylrest oder einen einfach mit Chlor substituierten $C_2$-$C_3$-Alkenylrest; $R_2$ und $R_3$ Halogen; und $R_4$ Wasserstoff oder Halogen bedeuten.

Wegen ihrer Wirkung als Schädlingsbekämpfungsmittel bevorzugt sind erfindungsgemässe Verbindungen der Formel I, die dadurch gekennzeichnet sind, dass $R_1$ einen $C_3$-Alkenylrest oder einen einfach mit Chlor substituierten $C_3$-Alkenylrest; $R_2$ Fluor, Chlor oder Brom; $R_3$ Fluor oder Chlor; und $R_4$ Wasserstoff, Fluor oder Chlor bedeuten.

Von besonderem Interesse sind ferner Verbindungen der Formel I, worin $R_1$ einen der Reste $CH_2=CH-CH_2-$ oder $Cl-CH=CH-CH_2-$ bedeutet.

Hervorzuheben sind auch Verbindungen der Formel I, worin $R_2$ Chlor oder Brom und/oder $R_3$ und $R_4$ Fluor bedeuten.

Die Verbindungen der Formel I fallen, wo dies prinzipiell möglich ist, als cis/trans-Isomerengemische an. In diesem Sinne sind als Verbindungen der Formel I sowohl die cis- oder trans-Formen als auch die entsprechenden Isomerengemische zu verstehen. Ein Isomerengemisch kann z.B. mit Hilfe der bekannten chromatographischen Trennmethoden und anschliessender Eluierung in die isomeren Formen getrennt werden. Zur Synthese stereochemisch einheitlicher Verbindungen der Formel I verwendet man mit Vorteil stereochemisch einheitliche Ausgangsverbindungen der nachstehenden Formeln II und IV.

Die Verbindungen der Formel I können nach an sich bekannten Verfahren hergestellt werden (vgl. u.a. die deutschen Offenlegungsschriften Nr. 2.123.236, 2.601.780).

So kann man z.B. eine Verbindung der Formel I erhalten durch Umsetzung

a) einer Verbindung der Formel II

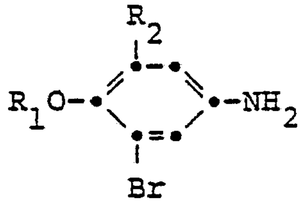

(II)

mit einer Verbindung der Formel III

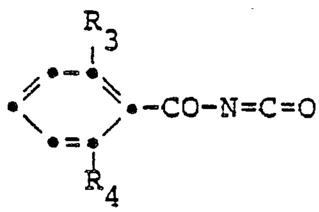

(III)

oder

b) einer Verbindung der Formel IV

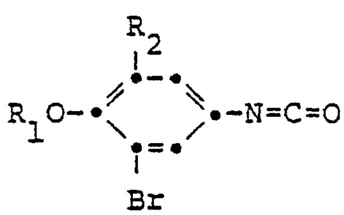

(IV)

gegebenenfalls in Gegenwart einer basischen Substanz mit einer Verbindung der Formel V

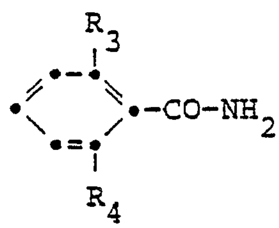

(V) .

In den obigen Formeln II, III, IV und V haben die Reste $R_1$, $R_2$, $R_3$ und $R_4$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther , Dipropyläther, Dibutyläther, Dioxan, Dimethoxy-

äthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Methyläthylketon, Methylisopropylketon und Methylisobutylketon. Verfahren a) wird im allgemeinen bei einer Temperatur von -10 bis 100°C, vorzugsweise zwischen 15 und 25°C, gegebenenfalls in Gegenwart einer organischen Base, z.B. Triäthylamin, durchgeführt. Die Durchführung von Verfahren b) erfolgt bei einer Temperatur von 0 bis 120°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden.

Es ist bereits bekannt, dass bestimmte N-Phenyl-N'-benzoylharnstoffe insektizide Eigenschaften besitzen (vgl. deutsche Offenlegungsschrift 2.123.236, 2.504.982, 2.537.413, 2.601.780 und 2.726.684, die belgischen Patentschriften 832.304, 843.906 und 844.066 sowie die US-Patentschrift 4.089.975).

Ueberraschenderweise wurde nun gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Hymenoptera, Anoplura, Siphonaptera, Mallophaga, Thysanura, Isoptera und Psocoptera.

Neben ihrer sehr günstig Wirkung gegenüber Fliegen, wie z.B. Musca domestica, und Mückenlarven eignen sich Verbindungen der Formel I auch zur Bekämpfung von pflanzenschädigenden Frassinsekten, in Zier- und Nutzpflanzen, insbesondere in Baumwollkulturen (z.B. gegen Spodoptera littoralis und Heliothis virescens) sowie in Gemüsekulturen (z.B. gegen Leptionatarsa decemlineata und Myzus persicae). Die Verbindungen der Formel I besitzen zum Teil ausgeprägte larvizide und/oder ovizide Wirkung. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z.B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:

organische Phosphorverbindungen, Nitrophenole und Derivaten, Formamidine, Harnstoffe, Carbamate und chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Träger und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind "cattle dips", d.h. Viehbäder, und "spray races", d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate; Spritzpulver (wettable powders), Pasten, Emulsionen;

b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

<u>Stäubemittel:</u> Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit ~~den~~ Trägerstoffen vermischt und vermahlen.

<u>Granulat:</u> Zur Herstellung eines 5%igen Granulates werden die folgenden Stoffe verwendet:

5 Teile Wirkstoff,
0,25 Teile epoxidiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz wird mit epoxidiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25,0 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1:1),

|  |  |  |
|---|---|---|
|  | 8,3 | Teile Natriumaluminiumsilikat, |
|  | 16,5 | Teile Kieselgur, |
|  | 46 | Teile Kaolin; |
| d) | 10 | Teile Wirkstoff, |
|  | 3 | Teile Gemisch der Natriumsalze von gesättigten Fettalkoholsulfaten, |
|  | 5 | Teile Naphthalinsulfonsäure/Formaldehyd-Kondensat, |
|  | 82 | Teile Kaolin. |

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen b) 25%igen und c) 50%igen emulgierbaren Konzentrates werden folgende Stoffe verwendet:

|  |  |  |
|---|---|---|
| a) | 10 | Teile Wirkstoff, |
|  | 3,4 | Teile epoxydiertes Pflanzenöl, |
|  | 3,4 | Teile eines Kombinationsemulgators, bestehend aus Fettalkoholpolyglykoläther und Alkylaralkyl-sulfonat-Calcium-Salz, |
|  | 40 | Teile Dimethylformamid, |
|  | 43,2 | Teile Xylol; |
| b) | 25 | Teile Wirkstoff, |
|  | 2,5 | Teile epoxydiertes Pflanzenöl, |
|  | 10 | Teile eines Alkylarylsulfonat/Fettalkoholpolyglykoläther-Gemisches, |
|  | 5 | Teile Dimethylformamid, |
|  | 57,5 | Teile Xylol; |

c) 50 Teile Wirkstoff,
4,2 Teile Tributylphenol-Polyglykoläther,
5,8 Teile Calcium-Dodecylbenzolsulfonat,
20 Teile Cyclohexanon,
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5%igen und b) 95%-igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);
b) 95 Teile Wirkstoff,
5 Teile epoxydiertes Pflanzenöl.

Beispiel 1: Zu einer Lösung von 5,2 g 3-Chlor-4-allyloxy-5-brom-anilin in 25 ml absolutem Aether werden bei Raumtemperatur unter Ausschluss von Feuchtigkeit 3,8 g 2,6-Difluorbenzoylisocyanat zugesetzt. Der ausfallende Niederschlag wird nach 1 Stunde abgesaugt und mit Aether gewaschen. Durch Umkristallisation aus Toluol erhält man N-(3-Chlor-4-allyloxy-5-brom)-phenyl-N'-difluorbenzoylharnstoff vom Schmelzpunkt 169-171°C.

Analog den vorstehend beschriebenen Arbeitsweisen werden die folgenden Verbindungen der Formel I hergestellt:

| $R_1$ | $R_2$ | $R_3$ | $R_4$ | Schmelzpunkt [°C] |
|---|---|---|---|---|
| $H_2C=CH-CH_2-$ | Cl | F | F | 169-171 |
| $H_2C=CH-CH_2-$ | Cl | F | Cl | 199-201 |
| $H_2C=CH-CH_2-$ | Cl | Cl | Cl | 219-221 |
| $H_2C=CH-CH_2$ | Cl | Cl | H | 172-173 |
| $H_2C=CH-CH_2-$ | Cl | F | H | 160-161 |
| $H_2C=CH-CH_2-$ | Cl | Cl | F | |
| $H_2C=CH-CH_2-$ | Br | F | F | 179-180 |
| $H_2C=CH-CH_2-$ | Br | F | Cl | 206-207 |
| $H_2C=CH-CH_2-$ | Br | Cl | Cl | 227-229 |
| $H_2C=CH-CH_2-$ | Br | Cl | H | 169-171 |
| $H_2C=CH-CH_2-$ | Br | F | H | 173-175 |
| $ClCH=CH-CH_2-$ | Cl | F | F | 171-172 *) |
| $ClCH=CH-CH_2-$ | Cl | F | Cl | 166-168 *) |
| $ClCH=CH-CH_2-$ | Cl | Cl | Cl | *) |
| $ClCH=CH-CH_2-$ | Cl | Cl | H | 189-191 *) |
| $ClCH=CH-CH_2-$ | Cl | F | H | 179-180 *) |
| $ClCH=CH-CH_2-$ | Br | F | F | 182-183 *) |
| $ClCH=CH-CH_2-$ | Br | F | Cl | 161-163 *) |
| $ClCH=CH-CH_2-$ | Br | Cl | Cl | 192-194 *) |
| $ClCH=CH-CH_2-$ | Br | Cl | H | 179-182 *) |
| $ClCH=CH-CH_2-$ | Br | F | H | 173-177 *) |
| $ClCH=CH-CH_2-$ | F | F | F | 138-143.5*) |
| $CH_2=CCl-CH_2-$ | Br | F | F | 187-188 |

*) Stereoisomerengemisch

Beispiel 2: Wirkung gegen Musca domestica: Je 50 frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

Beispiel 3: Wirkung gegen Lucilia sericata: Zu 9 ml eines Zuchtmediums wurden bei 50°C 1 ml einer 0,5% Aktivsubstanz enthaltenden wässrigen Lösung gegeben. Nun wurden ca. 30 frisch geschlüpfte Lucilia sericata-Larven zum Zuchtmedium gegeben und nach 48 und 96 Stunden wurde die insektizide Wirkung durch Ermittlung der Abtötungsrate festgestellt.

Verbindungen gemäss Beispiel 1 zeigten in diesem Test gute Wirkung gegen Lucilia sericata.

Beispiel 4: Wirkung gegen Aëdes aegypti: Auf die Oberfläche von 150 ml Wasser, das sich in einem Behälter befindet, wurde so viel einer 0,1%igen acetonischen Lösung des Wirkstoffes pipettiert, dass Konzentrationen von je 10, 5 und 1 ppm erhalten wurden. Nach Verdunsten des Acetons wurde der Behälter mit 30-40 2-tägigen Aëdes-Larven beschickt. Nach 1, 2 und 5 Tagen wurde die Moralität geprüft.

Verbindungen gemäss Beispiel 1 zeigten in diesem Test gute Wirkung gegen Aëdes aegypti.

Beispiel 5: Insektizide Frassgift-Wirkung: Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven im dritten larvalen Stadium besetzt. Der Versuch wurde bei 24°C und 60% relativer Luftfeuchtigkeit durchgeführt.

Verbindungen gemäss Beispiel 1 zeigten im obigen Test gute insektizide Frassgift-Wirkung gegen Spodoptera- und Heliothis-Larven.

Beispiel 6: Ovizide Wirkung auf Spodoptera littoralis: Auf Filterpapier abgelegte Eier von Spodoptera littoralis wurden aus dem Papier ausgeschnitten und in eine 0,05% Gew.-%ige Lösung des Wirkstoffes in einem Aceton-Wasser-Gemisch (1:1)getaucht. Die so behandelten Eiablagen wurden dann aus diesem Gemisch herausgenommen und bei 21°C und 60% relativer Feuchtigkeit in Kunststoffschalen deponiert.

Nach 3 bis 4 Tagen wurde die Schlupfrate, d.h. die Anzahl der Larven, die sich aus den behandelten Eiern entwickelt hatten, bestimmt.

Verbindungen gemäss Beispiel 1 zeigten gute Wirkung in obigen Test.

Beispiel 7: Wirkung auf Laspeyresia pomonella (Eier):

Abgelegte Eier von Laspeyresia pomonella, die nicht älter als 24 Stunden waren, wurden auf Filterpapier für 1 Minute in eine acetonisch-wässrige Lösung enthaltend 400 ppm des zu prüfenden Wirkstoffes eingetaucht. Nach den Antrocknen der Lösung wurden die Eier in Petrischalen gebracht und bei einer Temperatur von 28°C belassen. Nach 6 Tagen wurde die prozentuale Schlupfrate aus den behandelten Eiern bewertet.

Verbindungen gemäss Beispiel 1 zeigten gute Wirkung in obigem Test.

Beispiel 8: Wirkung auf Spodoptera littoralis und Heliothis virescens (Larven und Eier):

Es wurden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages wurden die eingetopften Pflanzen in ein Blechgefäss von etwa 20 Litern Inhalt gestellt, das mit einer Glasplatte abgedeckt wurde. Die Feuchtigkeit im Inneren des abgedeckten Gefässes wurde so reguliert, dass sich kein Kondenswasser bildete. Direktes, auf die Pflanzen fallendes Licht wurde vermieden. Dann wurden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens. Dazu wurden je 2 Blätter einer Pflanze in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis wurden zu den eingeschlossenen Blättern gegeben.

Nach 4 bis 5 Tagen erfolgte die Auswertung gegenüber unbehandelten Kontrollen unter Berücksichtigung folgender Kriterien:

a) Anzahl der noch lebenden Larven,

b) Larvale Entwicklungs- und Häutungshemmung,

c) Frasschaden (Schabfrass und Lochfrass),

d) Schlupfrate (Anzahl der aus den Eiern geschlüpften Larven).

Die Verbindungen gemäss Beispiel 1 zeigten gute Gesamt-Wirksamkeit in obigem Test.

Patentansprüche

1. Verbindung der Formel I

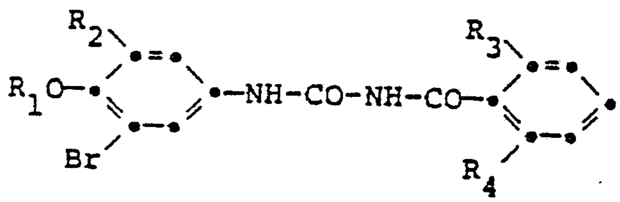

(I),

worin $R_1$ einen $C_2$-$C_3$-Alkenylrest oder einen einfach mit Chlor substituierten $C_2$-$C_3$-Alkenylrest; $R_2$ und $R_3$ Halogen; und $R_4$ Wasserstoff oder Halogen bedeuten.

2. Verbindung der Formel I gemäss Anspruch 1, dadurch gekennzeichnet, dass $R_1$ einen $C_3$-Alkenylrest oder einen einfach mit Chlor substituierten $C_3$-Alkenylrest; $R_2$ Fluor, Chlor oder Brom; $R_3$ Fluor oder Chlor; und $R_4$ Wasserstoff, Fluor oder Chlor bedeuten.

3. Verbindung der Formel I gemäss Anspruch 1 oder 2, dadurch gekennzeichnet, dass $R_1$ einen der Reste

$$CH_2=CH-CH_2- \quad \text{oder} \quad Cl-CH=CH-CH_2-$$

bedeutet.

4. Verbindung der Formel I gemäss Anspruch 1 bis 3, dadurch gekennzeichnet, dass $R_2$ Chlor oder Brom bedeutet.

5. Verbindung der Formel I gemäss Anspruch 1 bis 4, dadurch gekennzeichnet, dass $R_3$ und $R_4$ Fluor bedeuten.

6. Verbindung gemäss Anspruch 5 der Formel

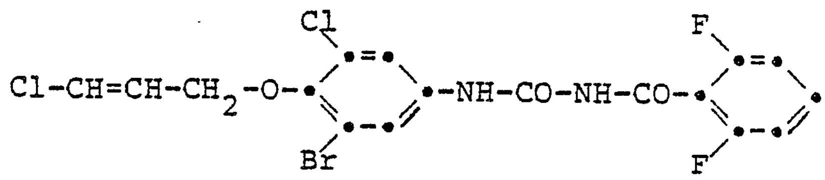

.

7. Verbindung gemäss Anspruch 5 der Formel

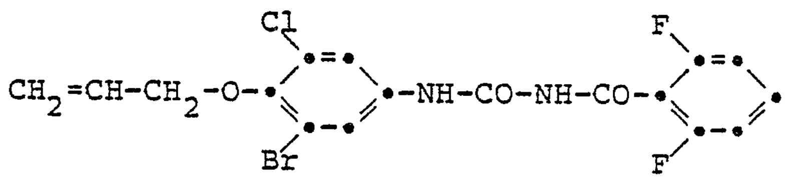

.

8. Verfahren zur Herstellung einer Verbindung gemäss den Ansprüchen 1 bis 7, dadurch gekennzeichnet, dass man

a) eine Verbindung der Formel II

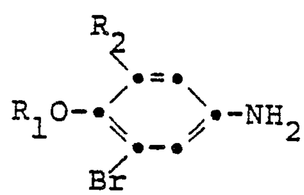

(II)

mit einer Verbindung der Formel III

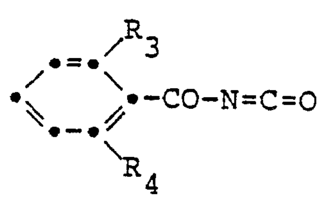

(III)

oder

b) eine Verbindung der Formel IV

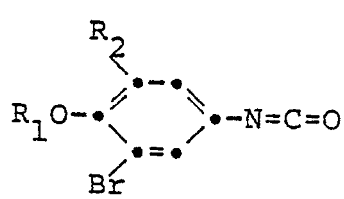

(IV)

mit einer Verbindung der Formel V

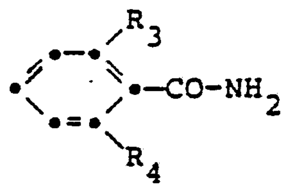

(V)

umsetzt, wobei in den Formeln II bis V die Reste $R_1$, $R_2$, $R_3$ und $R_4$ die in der Ansprüchen 1 bis 5 angegebenen Bedeutungen haben.

9. Schädlingsbekämpfungsmittel, welches als aktive Komponente eine Verbindung gemäss den Ansprüchen 1 bis 7 zusammen mit geeigneten Trägern und/oder anderen Zuschlagstoffen enthält.

10. Verwendung einer Verbindung gemäss den Ansprüchen 1 bis 7 zur Bekämpfung von Schädlingen, vorzugsweise von Insekten.